# EUROPEAN PATENT SPECIFICATION

(11) **EP 1 574 564 B1**
(45) Date of publication and mention of the grant of the patent: **22.07.2009**
(21) Application number: 03777359.5
(22) Date of filing: 08.12.2003
(51) Int. Cl.: C12N 1/02, C12Q 1/68

(54) **MICROORGANISM OR CELL COLLECTING METHOD, AND MICROORGANISM OR CELL COLLECTING IMPLEMENT USED FOR THE METHOD**
MIKROORGANISMUS- ODER ZELLSAMMELVERFAHREN UND FÜR DAS VERFAHREN VERWENDETES MIKROORGANISMUS- ODER ZELLENSAMMELGERÄT
PROCEDE DE PRELEVEMENT DE MICRO-ORGANISMES OU DE CELLULES ET OUTIL DE PRELEVEMENT DE MICRO-ORGANISMES OU DE CELLULES UTILISE POUR LEDIT PROCEDE

(30) Priority: 17.12.2002 JP 2002365818
(43) Date of publication of application: 14.09.2005
(73) Proprietor: ARKRAY, Inc., Minami-ku Kyoto-shi Kyoto 601-8045 (JP)
(72) Inventor: OKAMOTO, Masashi, c/o ARKRAY, Inc., Kyoto-shi, Kyoto 601-8045 (JP); KAMATA, Tatsuo, c/o ARKRAY, Inc., Kyoto-shi, Kyoto 601-8045 (JP); IZUMIZAWA, Yuji, c/o ARKRAY, Inc., Kyoto-shi, Kyoto 601-8045 (JP); MURAKAMI, Atsushi, c/o ARKRAY, Inc., Kyoto-shi, Kyoto 601-8045 (JP)
(74) Representative: Grant, Anne Rosemary
(86) International application number: PCT/JP2003/015686
(87) International publication number: WO 2004/055170

(56) References cited:
- JP-A- 7 111 887
- JP-A- 7 116 679
- JP-A- 49 041 577
- JP-A- 2000 014 380
- JP-A- 2001 112 497
- US-A- 5 085 781
- US-A- 6 010 869
- LUCORE L A ET AL: "Immobilization with metal hydroxides as a means to concentrate food-borne bacteria for detection by cultural and molecular methods." APPLIED AND ENVIRONMENTAL MICROBIOLOGY. MAY 2000, vol. 66, no. 5, May 2000 (2000-05), pages 1769-1776, XP002352194 ISSN: 0099-2240

## Description

### Technical Field

The present invention relates to a method of collecting microorganisms or cells from a liquid sample and to a method of performing gene amplification or gene detection using the same.

### Background Art

Tuberculosis still is a serious bacterial disease worldwide, and not only treatment methods but also diagnostic methods therefor are extremely important. A final diagnosis as to the tuberculosis infection is made by carrying out a culture method. However, since bacteria responsible for tuberculosis (hereinafter referred to as "TB bacteria") have an extremely slow growth rate, the establishment of a preliminary diagnostic method to be performed prior to the culture method has been desired. As such a preliminary diagnostic method, a genetic analysis method using a polymerase chain reaction (PCR) method is attracting attention. In this method, a primer specific to a gene of TB bacteria is used to amplify a gene of the TB bacteria, if any, so that it can be detected, thereby enabling the presence or absence of the TB bacteria to be determined.

In the above-described preliminary diagnostic method using the PCR method, collecting TB bacteria from a liquid sample such as sputum is necessary as a pretreatment. Conventionally, the collection of the TB bacteria has been carried out in the following manner, for example ("New Guideline for TB Bacteria Test 2000" published by Japan Anti-Tuberculosis Association, pp. 28-29). First, a solution (a NALC-NaOH solution) containing N-acetyl-L-cysteine (NALC) and sodium hydroxide (NaOH) is added to the liquid sample to remove the viscosity therefrom, thus preparing a sample solution. The sample solution is centrifuged at 13000 g for 10 minutes, and the precipitated TB bacteria are collected. However, the conventional collection method using centrifugation has problems that the conditions for performing the centrifugation are severe, the operation for performing the centrifugation is complicated, and besides, the centrifugation takes a long time and requires the use of an expensive centrifuge. Also, there have been problems in conventional methods of lysing the TB bacteria and amplifying or detecting genes of the TB bacteria to be performed subsequent to the collection of the TB bacteria. Examples of conventional lysis methods include chemical methods using organic solvents or the like and physical methods using ultrasonic waves or repeating freezing and thawing. However, since TB bacteria have a high cell-wall lipid content, such conventional lysis methods cannot extract genes sufficiently. In order to achieve sufficient gene extraction, it is necessary to make the treatment conditions more severe, which requires the use of a special device and/or a special reagent. Besides, this may result in a longer treatment period and a more complicated operation. Such problems with regard to the collection, lysis, gene amplification, and the like relate to the entire acid-fast bacteria group including TB bacteria. These problems may occur in the case of other bacteria and viruses. Also, similar problems occur in the case of cells such as leukocytes.

In this context it is noted that JP-A-711 887 discloses a method of preservation of microorganisms (among which Mycobacteria) contained in fluid, on a water-absorbent resin (AKUA rucksack). Moreover disclose Lucore et al. (May 2000, Applied and Environmental Microbiology, vol. 66(5), pp. 1769-1776) the immobilisation with metal hydroxydes as means to concentrate food borne bacteria, such as Listeria monocytogenes or Salmonella enterica, for detection by cultural and molecular methods.

On the other hand, in order to measure the concentration of legionella bacteria, which recently are perceived as a problem, present in baths, swimming pools, and the like or the concentration of *Escherichia coli* or the like present in rivers, lakes, seas, and the like, it is necessary to collect several liters to several tons of test sample and collect microorganisms or the like from this test sample. However, it is difficult to achieve efficient collection by conventional methods, and the development of a technique enabling the efficient collection of bacteria or the like from a large amount of sample has been desired.

### Disclosure of Invention

Therefore, with the foregoing in mind, it is a first object of the present invention to provide a method of collecting microorganisms or cells easily and in a short time as set out in claim 1. It is a second object of the present invention to provide a method of specifically amplifying or detecting a gene, by which microorganisms or cells can be detected easily and in a short time as set out in claim 12.

In order to achieve the first object, the present invention provides a method of collecting a microorganism or a cell from a liquid sample, including bringing the liquid sample into contact with water-absorbing resin so that a liquid phase part of the liquid sample is absorbed by the water-absorbing resin and the microorganism or the cell is caught on a surface of the water-absorbing resin.

According to the method of the present-invention, the microorganism or the cell can be caught by the water-absorbing resin easily in a short time. Thus, centrifugation that requires severe treatment conditions need not be performed. Moreover, it is possible to design the water-absorbing resin to have a required size, amount, or the like in accordance with the amount of test sample. As a result, even when the amount of the test sample is as great as several liters to several tons, target bacteria (e.g., legionella bacteria or *Escherichia coli)* or the like can be collected merely by bringing the test sample into contact with the water-absorbing resin.

Next, in order to achieve the second object, the present invention provides a method of amplifying or detecting specifically a gene of a microorganism or a cell, including: collecting a microorganism or a cell by the method of the present invention; extracting a gene of the microorganism or the cell by adding an extraction reagent solution containing a nonionic detergent to the microorganism or the cell and heating the resultant mixture; and amplifying or detecting specifically the extracted gene.

This method employs the collection method of the present invention. According to this method, gene extraction can be achieved merely by adding the extraction reagent solution containing the nonionic detergent to the microorganism or the cell and heating the resultant mixture. This allows a quick shift to the subsequent operation such as gene amplification.

### Brief Description of Drawings

FIG. 1 is a cross-sectional view showing an example of the configuration of a centrifugation tube to be used in a collection method according to the present invention.
FIGs. 2A to 2D illustrate major processes of an example of a collection method according to the present invention.

### Best Mode for Carrying Out the Invention

Hereinafter, the present invention will be described more specifically

In the method of collecting a microorganism or a cell according to the present invention as set out in claim 1, it is preferable that, after the liquid sample has been brought into contact with the water-absorbing resin, a collecting solution further is brought into contact with the water-absorbing resin to collect the microorganism or the cell caught on the surface of the water-absorbing resin in the collecting solution. By repeating this collecting operation a plurality of times, the amount of the collecting solution added decreases gradually, thereby increasing the concentration of the microorganism or the cell in the collecting solution. This allows a subsequent process such as centrifugation to be performed more efficiently. The collecting solution is not particularly limited, and may be, for example, water, distilled water, ion exchanged water, ultrapure water, a buffer, or the like. However, it is preferable that an extraction solution for gene extraction of the microorganism or the cell is used as the collecting solution, as will be described later.

The amount of the liquid sample added preferably is not greater than a water-absorbing capacity of the water-absorbing resin. Also, it is possible to change the amount of the water-absorbing resin in accordance with the amount of the liquid sample to be added so that the amount of the liquid sample to be added would not be greater than a water-absorbing capacity of the water-absorbing resin. Furthermore, the amount of the collecting solution added preferably is greater than a water-absorbing capacity of the water-absorbing resin that has absorbed the liquid phase part of the liquid sample. It is preferable that the water-absorbing resin has a water-absorbing capacity of, for example, 2 to 10000 times its own weight, preferably 5 to 5000 times its own weight, and more preferably 10 to 1000 times its own weight. When treating a large amount of test sample, the treatment preferably is performed using water-absorbing resin with a large water-absorbing capacity, e.g., the one with a water-absorbing capacity of 10000 times its own weight. The water-absorbing resin is not particularly limited, and may be, for example, a hydrophilic cross-linked polymer having a hydrophilic functional group. Examples of the hydrophilic functional group include anionic, nonionic, and cationic hydrophilic functional groups, such as a carboxyl group, a sulfonyl group, and an amino group. Specific examples of the water-absorbing resin include crosslinking substances of carboxymethyl cellulose, hydrolysates of starch-acrylonitrile graft polymer, neutralized substances of starch-acrylic acid graft polymer, crosslinking substances of polyacrylate polymer, crosslinking substances of polymethacrylate polymer, saponified substances of vinyl acetate-acrylic ester copolymer, hydrolysates of acrylonitrile polymer or acrylamide copolymer or crosslinking substances thereof, crosslinking substances of polymers containing a sulfone group, and crosslinking substances of polyethylene oxide or polyethyleneimine. They may be used either alone or in combinations of at least two kinds thereof. Among these, crosslinking substances of partially neutralized polyacrylate or polyacrylic acid are preferable, and polyacrylate is more preferable. Specific examples of polyacrylate include sodium polyacrylate. The form of the water-absorbing resin is not particularly limited, but it is preferable that the water-absorbing resin is provided as particles, as will be described later.

In the method of collecting a microorganism or a cell according to the present invention, it is preferable that a centrifugation tube including a filter that divides an inner space of the centrifugation tube into an upper part and a lower part and water-absorbing resin particles disposed on the filter is provided, the liquid sample is poured into the centrifugation tube to bring the liquid sample into contact with the water-absorbing resin particles, the collecting solution is then poured into the centrifugation tube to bring the collecting solution into contact with the water-absorbing resin particles, and the centrifugation tube is centrifuged so that the collecting solution passes through the filter to move toward a bottom of the centrifugation tube.

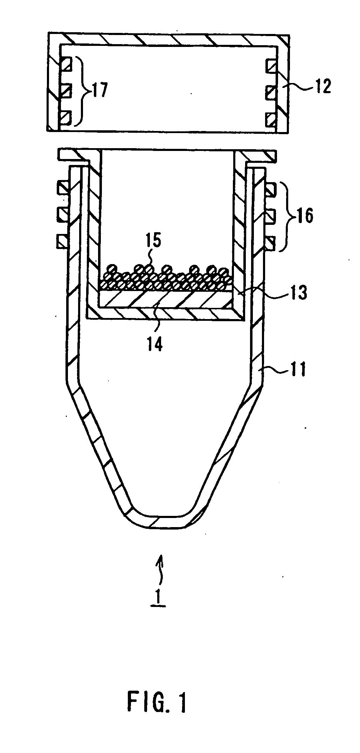

The centrifugation tube used in the above-described method is a centrifugation tube including a filter that divides an inner space of the tube into an upper part and a lower part and water-absorbing resin particles disposed on the filter. FIG. 1 shows an example of such a centrifugation tube. As shown in FIG. 1, main components of this centrifugation tube are a tube body 11 and a cap 12. A screw thread 16 is formed on an upper part of an outer wall of the tube body 11, while a thread groove 17 is formed on an inner wall of the cap 12. Thus, the tube body 11 and the cap 12 are configured so as to be screwed together. A supporter 13 in the shape of a cylinder with a bottom is inserted into the tube body 11. On the bottom of the supporter 13, a filter 14 is disposed, and water-absorbing resin particles 15 are disposed on this filter 14. The filter is not particularly limited, and the pore size thereof may be, for example, in the range from 10 nm to 100 µm, preferably from 0.1 to 20 µm, and more preferably from 0.1 to 10 µm. The material for the filter is not particularly limited, and may be, for example, polyvinylidene fluoride, cellulose nitrate, hydrophilic polyethersulfone, polytetrafluoroethylene, polycarbonate, polyamide, polysulfone, polyethylene, polypropylene, polymer alloy, acetylcellulose, or the like. Among these, polyvinylidene fluoride and polycarbonate are preferable, and polycarbonate is more preferable.

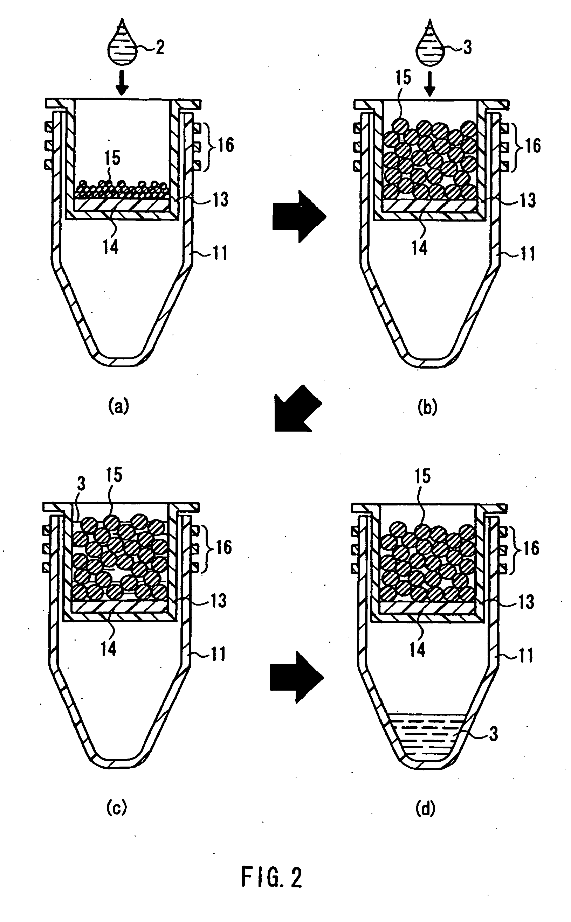

FIGs. 2A to 2D illustrate an example of a method of collecting microorganisms or cells using this centrifugation tube 1. It is to be noted that portions in common between FIG. 1 and FIGs. 2A to 2D are numbered identically. First, as shown in FIG. 2A, a liquid sample 2 is dropped into a tube body 11. Water-absorbing resin particles 15 then swell by absorbing a liquid phase part of the liquid sample 2, as shown in FIG. 2B. The amount of the liquid sample 2 dropped at this time is not greater than the water-absorbing capacity of the water-absorbing resin particles 15 as a whole, and microorganisms or cells adhere to surfaces of the water-absorbing resin particles 15. Thereafter, as shown in FIG. 2B, a collecting solution 3 further is dropped onto the water-absorbing resin particles 15. As described above, it is preferable to use an extraction reagent solution as the collecting solution. The amount of the collecting solution 3 dropped is greater than the water-absorbing capacity of the water-absorbing resin particles 15 that have absorbed the liquid phase part of the liquid sample 2 as a whole. Thus, as shown in FIG. 2C, the collecting solution 3 that has not been absorbed by the water-absorbing resin particles 15 accumulates in a supporter 13, and the microorganisms or the cells are collected in the accumulating collecting solution 3. At this time, in order to improve the collection efficiency of the microorganisms or the cells, it is preferable to cause the water-absorbing resin particles 15 to shake well in the collecting solution 3 by, for example, repeated pipetting. Thereafter, the tube body 11 is capped with a cap 12 (not shown) and then is centrifuged. In the present invention, the conditions for the centrifugation are not particularly limited. For example, the centrifugation may be performed at 500 to 13000 g for 3 seconds to 60 minutes, preferably at 1000 to 10000 g for 10 seconds to 10 minutes, and more preferably at 5000 g for 1 minute. The centrifuge to be used is not particularly limited, and may be, for example, a desktop centrifuge. After the centrifugation, the collecting solution accumulates in the lower part of the centrifugation tube, as shown in FIG. 2D. This collecting solution is collected and used as a sample in a genetic analysis that will be described

Next, in the method of amplifying or detecting a gene according to the present invention as set out in claim 12, the extraction reagent solution preferably is used as the present invention the extraction reagent solution preferably is used as the collecting solution in the above-described method of collecting microorganisms or cells. When the collecting solution is the extraction reagent solution, the gene can be extracted by heating the collecting solution simply as it is. This allows the operation for adding an extraction reagent solution to be omitted. The heating temperature of the extraction reagent solution is not particularly limited, but preferably is not lower than 70°C and lower than 100°C. By setting the heating temperature to a temperature lower than 100°C, the following advantages can be obtained, for example: there is reduced concern that a sample might be scattered, and the temperature can be controlled easily so that a special heater is not required. The heating temperature preferably is not lower than 80°C and lower than 100°C, and most suitably 96°C. Furthermore, the heating is performed, for example, for 1 to 30 minutes, preferably for 10 minutes. The pH of the liquid is, for example, in the range from 7.0 to 12.0, preferably 8.0. The concentration of the nonionic detergent in the extraction reagent solution is, for example, 0.01 to 10 wt%, preferably 0.5 to 2.0 wt%, and more preferably 1.0 wt%.

Examples of the nonionic detergent include: D-sorbitol fatty acid esters such as Span 20, Span 40, Span 60, Span 65, Span 80, and Span 85 (all manufactured by Nacalai Tesque, Inc., for example); polyoxyethyleneglycol sorbitan alkyl esters such as Tween 20, Tween 21, Tween 40, Tween 60, Tween 65, Tween 80, Tween 81, and Tween 85 (all manufactured by Nacalai Tesque, Inc., for example); polyoxyethyleneglycol p-t-octylphenyl ethers such as Triton X-100 (manufactured by Nacalai Tesque, Inc., for example). These detergents may be used either alone or in combinations of at least two kinds thereof. Among these, Triton X-100, Tween 20, and Tween 21 are preferable, and Triton X-100 is more preferable.

In the method of amplifying or detecting a gene according to the present invention, it is preferable that the extraction reagent solution further contains a metal chelating agent. The metal chelating agent serves to prevent a gene-degrading enzyme such as DNase contained in a sample from degrading the gene, for example. The concentration of the metal chelating agent in the extraction reagent solution is, for example, 0.1 to 100 mM, preferably 1.0 mM. Examples of the metal chelating agent include ethylenediaminetetraacetic acid (EDTA), ethylene glycol bis(β-aminoethyl ether)-N,N,N',N'-tetraacetic acid (EGTA), diaminocyclohexane tetraacetic acid, o-phenanthroline, and salicylic acid. These metal chelating agents may be used either alone or in combinations of at least two kinds thereof. Among these, EDTA and EGTA are preferable, and EDTA is more preferable.

The microorganism to which the present invention is applicable is not particularly limited. Examples thereof include acid-fast bacteria, atypical mycobacteria, gonococcus, legionella bacteria, mycoplasmas, spirochetes, syphilis spirochetes, chlamydiae, rickettsiae, *Mycobacterium leprae,* Spirillum minus, staphylococci, streptococci, *Escherichia coli, Pseudomonas aeruginosa, Pasteurella pestis,* viruses, Japanese encephalitis virus, hepatitis B virus, hepatitis C virus, ATLV, HIV, and Ebola virus. Examples of the acid-fast bacteria include *M. avium, M. intracellularae, M. gordonae, M*. *tuberculosis, M. kansasii, M. fortuitum, M. chelonae, M. bovis, M. scrofulaceum, M. paratuberculosis, M. phlei, M. marinum, M. simiae, M. scrofulaceum, M. szulgai, M. leprae, M. xenopi, M. ulcerans, M. lepraemurium, M. flavescens, M. terrae, M nonchromogenicum, M. malmoense, M. asiaticum, M. vaccae, M. gastri, M. triviale, M. haemophilum, M. africanum, M. thermoresistable,* and *M. smegmatis.* Furthermore, the cell to which the present invention is applicable is not particularly limited. Examples thereof include cellular tissues containing a genetic material, such as human leukocytes, human somatic cells, or cells of other animals and plants.

In the present invention, the liquid sample may be, for example, sputum, spinal fluid, feces, saliva, blood, a tissue, urine, or a sample obtained by pretreating any one of these biological samples. Examples of the pretreated sample include a sample obtained by treating sputum with N-acetyl-L-cysteine (NALC) and sodium hydroxide (NaOH). Other than these, water in baths, water in swimming pools, water in fish farms, water in rivers, lake water, seawater, and the like also may be used as the liquid sample in the present invention.

The amount of the liquid sample in the present invention is not particularly limited. However, when a relatively small amount of sample is required as in the case of a clinical test or the like, the amount of the liquid sample is, for example, in the range from 5 µL to 10 mL, preferably from 10 µL to 1 mL, and more preferably from 50 µL to 500 µL. On the other hand, when a relatively large amount of sample is used, for example, as in the case where the concentration of microorganisms or the like in water in a bath, a river, or the like is measured as part of water examination, the amount of the liquid sample should be within the range determined by ministerial ordinances given by the respective government agencies, for example. More specifically, the amount of the liquid sample is in the range from 1 mL to 10 L, preferably from 10 mL to 1 L, and more preferably from 50 mL to 200 mL. Examples of the ministerial ordinances include "Manual of Prevention Measures against Legionella Infection in Circulating Type Bath" (issued by the director of the Environmental Health Division of the Health Service Bureau in the Ministry of Health, Labour and Welfare in Japan as a 95th notification dated September 11, 2001). The manual describes, for example, legionella bacteria in a bath should be less than 10 CFU per 100 mL. Thus, when detecting legionella bacteria in a bath, it is necessary to use at least 100 mL of sample. It is to be noted here that the amount of the liquid sample preferably does not exceed the water-absorbing capacity of the water-absorbing resin, as described above.

Next, the method of amplifying or detecting a gene according to the present invention can be carried out in the following manner, for example. First, to a buffer having a pH in the above-described predetermined range, a metal chelating agent such as EDTA is added as necessary and a nonionic detergent further is added, thus preparing an extraction reagent solution. As the buffer, Tris-HCl buffer, HEPES buffer, MOPS buffer, HEPPS buffer, TAPS buffer, phosphate buffer, or the like may be used. This extraction reagent solution preferably is sterilized by high-pressure steam in an autoclave. On the other hand, a sample solution is prepared. For example, a sputum specimen is homogenized and sterilized by the N-acetyl-L-cysteine-NaOH method (NALC-NaOH method) or the like. The thus-treated specimen (a liquid sample) is added to a centrifugation tube (e.g., the one shown in FIG. 1) that includes a collection filter inside and water-absorbing resin particles disposed on the collection filter, and the extraction reagent solution (a collecting solution) further is added to the centrifugation tube. The centrifugation tube is then centrifuged (e.g., 5000 g, 1 minute) so that the extraction reagent solution accumulates in the lower part of the centrifugation tube. An extraction treatment is then performed by heating the extraction reagent solution that has accumulated, either as it is or after being transferred into a different tube, at a temperature in the above-described predetermined range using a heating block or the like. Examples of heating means other than the heating block include a water bath, a microwave oven, and an air bath. The specimen that has subjected to the above-described extraction treatment may be subjected to a gene amplification treatment or a gene detection treatment simply as it is or after being pretreated. Examples of the method of performing the gene amplification or gene detection include a PCR method and modifications of the PCR method, such as a RT-PCR method. Furthermore, examples of the gene to be analyzed include DNA and RNA. In the case where the collecting solution is different from the extraction reagent solution, the extraction reagent solution is added to the collecting solution to carry out the extraction treatment.

### EXAMPLES

A genetic analysis was carried out with regard to cultures of TB bacteria in the following manner.

### (Preparation of TB bacteria)

A clinical isolate of TB bacteria was cultured in a product named MycoBroth (Kyokuto Pharmaceutical Industrial Co., Ltd.) at 35°C until a turbidity corresponding to #1 of the McFarland turbidity standard was obtained. Then, the culture was diluted with 0.067 M phosphate buffer (pH: 6.8) so as to achieve a series of 10-fold dilutions (10⁰-fold to 10¹⁰⁻fold). The thus-obtained solutions were used as samples.

### (Operation for collecting TB bacteria)

Centrifugation tubes (see FIG. 1), each including a filter unit (see FIG. 1) provided with a product named Isopore Filter (pore size 3 µm, Millipore Corporation) and 0.0065 g of water-absorbing resin particles (product name "AQUA KEEP 10SH P", SUMITOMO SEIKA CHEMICALS CO.,LTD.) disposed on this filter unit, were provided. 100 µL of the above-described samples with the diluted TB bacteria were added to these centrifugation tubes, respectively. Thereafter, 500 µL of an extraction reagent solution further was added to each of the centrifugation tubes. This extraction reagent solution also served as a collecting solution and had been prepared by dissolving Triton X-100 (Nacalai Tesque, Inc.) in TE buffer (10 mM EDTA and 25 mM Tris-HCl, pH 8.0) so that its concentration became 1% and then autoclaving the mixture. The centrifugation tubes were then centrifuged at 5000 g for 1 minute, and the filtrates (about 100 µL) that accumulated in the bottom of the centrifugation tubes were collected.

### (Gene extraction)

The filtrates were heated at 96°C for 10 minutes in a heating block to lyse the TB bacteria.

### (Gene detection by PCR)

To 25 µl of each of the lysates, 50 µL of the master mixture of a product named AMPLICOR Amplification Kit (Nippon Roche K.K.) and 25 µL of 15 mM magnesium acetate were added to cause a PCR. Conditions and an operation for causing the PCR were pursuant to the document attached to the kit. Detection of a gene was carried out using a product named AMPLICOR Detection Kit (Nippon Roche K.K.). The operation for detecting the gene was carried out pursuant to the document attached to the kit.

### (Comparative Example 1)

As a comparative example, samples prepared in the same manner as in the above were treated using a product named AMPLICOR Pretreatment Kit (Nippon Roche K.K.). With regard to the thus-treated samples, gene detection by the PCR was carried out in the same manner as in the above.

### (Results)

In Example 1 and Comparative Example 1, the samples with a dilution factor of up to 10⁵ were judged as tuberculosis positive and those with a dilution factor of greater than 10⁵ were judged as tuberculosis negative. Furthermore, internal controls (ICs) were all judged as tuberculosis positive in both Example 1 and Comparative Example 1. Thus, it can be said that the effect of collecting and lysing the cultures of the TB bacteria achieved by the method of Example 1 of the present invention is equivalent to that achieved by the conventional method of Comparative Example 1. Moreover, from the fact that ICs were all judged as tuberculosis positive, it is understood that contamination of substances that interfere with the PCR was also suppressed. Besides, according to the method of Example 1 of the present invention, the time required for the collection and the lysis was reduced to about 1/5 of that in the method of Comparative Example 1.

### (Example 2)

Sputum specimens (7 specimens) collected from patients with suspected tuberculosis (TB) were treated by the NALC-NaOH method, thus preparing samples. Then, with regard to these samples, collection, gene extraction, and gene detection by the PCR were carried out in the same manner as in Example 1. The results are shown in Table 1 below.

### (Comparative Example 2)

Samples prepared in the same manner as in Example 2 were treated using a product named AMPLICOR Pretreatment Kit (Nippon Roche K.K.). With regard to the thus-treated samples, gene detection by the PCR was carried out in the same manner as in Example 1. The results are shown in Table 1 below.

**(Table 1)**

| Sample No. | Example* | Comparative Example* |
|---|---|---|
| 1 | 1.786 | 2.796 |
| 2 | 2.809 | 2.662 |
| 3 | 2.875 | 2.85 |
| 4 | 2.668 | 2.8 |
| 5 | 2.772 | 2.816 |
| 6 | 2.752 | 2.741 |
| 7 | 2.728 | 2.736 |

| | | |
|---|---|---|
| * absorbance (wavelength: 450 nm) | | |

As shown in Table 1, in both Example 2 and Comparative Example 2, all the 7 specimens were judged as tuberculosis positive, and internal controls (ICs) also were all judged as tuberculosis positive. Thus, it can be said that the method of the present invention can exhibit a similar performance to that of the conventional method. Moreover, the method of the present invention can perform the operations for collecting and lysing the TB bacteria more easily and more quickly than the conventional method.

### Industrial Applicability

As specifically described above, the present invention provides a method of collecting microorganisms or cells easily and in a short time without using any special device. Therefore, by applying the method of the present invention to, for example, a pretreatment of a sample to be analyzed in an examination of microorganisms or the like utilizing gene amplification and detection, higher efficiency of the examination can be realized easily.

## Claims

1. A method of collecting a microorganism or a cell from a liquid sample, comprising:
the provision of a centrifugation tube comprising a filter on which water-absorbing resin particles are disposed, said filter dividing an inner space of the tube into an upper part and a lower part, wherein the liquid sample is poured into the centrifugation tube to bring the liquid sample into contact with the water-absorbing resin so that a liquid phase part of the liquid sample is absorbed by the water-absorbing resin, and a collecting solution is then poured into the centrifugation tube to bring the collecting solution into contact with the water-absorbing resin to collect the microorganism or the cell caught on the surface of the water-absorbing resin in the collecting solution, and the centrifugation tube is centrifuged so that the collecting solution passes through the filter to move toward a bottom of the centrifugation tube.

2. The method according to claim 1, wherein the centrifugation is performed at 500 to 13000 g for 3 seconds to 60 minutes.

3. The method according to any one of claims 1 to 2, wherein an amount of the liquid sample added is not greater than a water-absorbing capacity of the water-absorbing resin.

4. The method according to any one of claims 1 to 3, wherein the amount of the collecting solution added is greater than a water-absorbing capacity of the water-absorbing resin that has absorbed the liquid phase part of the liquid sample.

5. The method according to any one of claims 1 to 4, wherein the water-absorbing resin is a hydrophilic cross-linked polymer having a hydrophilic functional group.

6. The method according to any one of claims 1 to 5, wherein the microorganism to be collected is at least one selected from the group consisting of acid-fast bacteria, atypical mycobacteria, gonococcus, legionella bacteria, mycoplasmas, spirochetes, syphilis spirochetes, chlamydiae, rickettsiae, *Mycobacterium leprae,* Spirillum minus, staphylococci, streptococci, *Escherichia coli, Pseudomonas aeruginosa, Pasteurella pestis,* viruses, Japanese encephalitis virus, hepatitis B virus, hepatitis C virus, ATLV, HIV, and Ebola virus.

7. The method according to claim 6, wherein the acid-fast bacterium is at least one selected from the group consisting of *M. avium, M intracellularae, M. gordonae, M tuberculosis, M. kansasii, M. fortuitum, M. chelonae, M bovis, M. scrofulaceum, M paratuberculosis, M. phlei, M. marinum, M. simiae, M. scrofulaceum, M. szulgai, M. leprae, M xenopi, M ulcerans, M. lepraemurium, M. flavescens, M. terrae, M nonchromogenicum, M. malmoense, M. asiaticum, M. vaccae, M. gastri, M. triviale, M. haemophilum, M. africanum, M. thermoresistable,* and *M. smegmatis.*

8. The method according to any one of claims 1 to 7, wherein the liquid sample is at least one selected from the group consisting of sputum, spinal fluid, feces, saliva, blood, tissues, swab, liquid obtained by gastrolavage, urine, samples obtained by pretreating these biological samples, water in baths, water in swimming pools, water in fish farms, water in rivers, lake water, and seawater.

9. The method according to any one of claims 1 to 8, wherein the amount of the liquid sample is in a range from 50 µL to 500 µL.

10. The method according to any one of claims 1 to 8, wherein the amount of the liquid sample is in a range from 50 mL to 200 mL.

11. An implement for collecting a microorganism or a cell used for the method according to claim 1, comprising a centrifugation tube,
the centrifugation tube comprising:
a filter that divides an inner space of the centrifugation tube into an upper part and a lower part; and
water-absorbing resin particles disposed on the filter.

12. A method of amplifying or detecting specifically a gene of a microorganism or a cell, comprising:
collecting a microorganism or a cell by the method according to any one of claims 1 to 10;
extracting a gene of the microorganism or the cell by adding an extraction reagent solution containing a nonionic detergent to the microorganism or the cell and heating the resultant mixture; and
amplifying or detecting specifically the extracted gene.

13. The method according to claim 12, wherein the extraction reagent solution also serves as the collecting solution.

14. The method according to claim 12 or 13, wherein the heating temperature is not lower than 70°C and lower than 100°C.

15. The method according to any one of claims 12 to 14, wherein the heating is performed for 1 to 30 minutes.

16. The method according to claim 12 or 13, wherein the heating is performed at 96°C for 10 minutes.

17. The method according to any one of claims 12 to 16, wherein a pH of the extraction reagent solution is in a range from 7.0 to 12.0.

18. The method according to any one of claims 12 to 17, wherein a concentration of the nonionic detergent in the extraction reagent solution is in a range from 0.01 to 10 wt%.

19. The method according to any one of claims 12 to 18, wherein the nonionic detergent is at least one selected from the group consisting of D-sorbitol fatty acid esters, polyoxyethyleneglycol sorbitan alkyl esters, and polyoxyethyleneglycol p-t-octylphenyl ethers.

20. The method according to any one of claims 12 to 19, wherein the extraction reagent solution further contains a metal chelating agent.

21. The method according to claim 20, wherein a concentration of the metal chelating agent in the extraction reagent solution is 0.1 to 100 mM.

22. The method according to claim 20 or 21, wherein the metal chelating agent is at least one selected from the group consisting of ethylenediaminetetraacetic acid (EDTA), ethylene glycol bis(β-aminoethyl ether)-N,N,N',N'-tetraacetic acid (EGTA), diaminocyclohexane tetraacetic acid, o-phenanthroline, and salicylic acid.

23. The method according to any one of claims 12 to 22, wherein the gene is amplified or detected specifically by a polymerase chain reaction (PCR) method.

## Patentansprüche

1. Verfahren zum Sammeln eines Mikroorganismus oder einer Zelle aus einer flüssigen Probe, wobei man:
ein Zentrifugationsrohr bereitstellt, umfassend einen Filter, auf welchem wasserabsorbierende Harzpartikel angebracht sind, wobei der Filter den Innenraum des Rohres in eine obere und eine untere Hälfte aufteilt, wobei die flüssige Probe in das Zentrifugationsrohr eingegossen wird, um die flüssige Probe mit dem wasserabsorbierenden Harz in Kontakt zu bringen, so dass ein Teil der flüssigen Phase der flüssigen Probe durch das wasserabsorbierende Harz absorbiert wird, und man dann eine Sammellösung in das Zentrifugationsrohr gießt, um die Sammellösung mit dem wasserabsorbierenden Harz in Kontakt bringt, um den Mikroorganismus oder die Zelle, die an der Oberfläche des wasserabsorbierenden Harzes zurückgehalten wird, in der Sammellösung zu sammeln, und man das Zentrifugationsrohr zentrifugiert, so dass die Sammellösung durch den Filter hindurchtritt und sich zum Boden des Zentrifugationsrohrs hin bewegt.

2. Verfahren nach Anspruch 1, wobei die Zentrifugation bei 500 bis 13.000 g über einen Zeitraum von 3 Sekunden bis 60 Minuten durchgeführt wird.

3. Verfahren nach einem der Ansprüche 1 und 2, wobei die Menge der zugegebenen flüssigen Probe nicht größer ist als die wasserabsorbierende Kapazität des wasserabsorbierenden Harzes.

4. Verfahren nach einem der Ansprüche 1 bis 3, wobei die Menge der zugesetzten Sammellösung größer als die wasserabsorbierende Kapazität des wasserabsorbierenden Harzes ist, das den Flüssigphasenanteil der flüssigen Probe adsorbiert hat.

5. Verfahren nach einem der Ansprüche 1 bis 4, wobei das wasserabsorbierende Harz ein hydrophiles, quervernetztes Polymer mit einer hydrophilen, funktionalen Gruppe ist.

6. Verfahren nach einem der Ansprüche 1 bis 5, wobei der zu sammelnde Mikroorganismus wenigstens ein Mikroorganismus ist, ausgewählt unter der Gruppe, bestehend aus säurebeständigen Bakterien, atypischen Mycobakterien, Gonokokken, Legionella-Bakterien, Mycoplasmen, Spirocheten, Syphilisspirocheten, Chlamydien, Rickettsiaen, *Mycobacterium leprae, Spirillum minus,* Staphylokokken, Streptokokken, *Escherichia coli, Pseudomonas aeruginosa, Pasteurella pestis,* Viren, Japanischem Encephalitisvirus, Hepatitis B-Virus, Hepatitis C-Virus, ATLV, HIV und Ebola-Virus.

7. Verfahren nach Anspruch 6, wobei das säurebeständige Bakterium wenigstens ein Bakterium ist, ausgewählt unter der Gruppe, bestehend aus *M*. *avium, M*. *intracellularae, M. gordonae, M. tuberculosis, M. kansasii, M. fortuitum, M. chelonae, M. bovis, M. scrofulaceum, M. paratuberculosis, M. phlei, M. marinum M. simiae, M. scrofulaceum, M. szulgai, M. leprae, M. xenopi, M. ulcerans, M. lepraemurium, M. flavescens, M. terrae, M. nonchromogenicum, M. malmoense, M. asiaticum, M. vaccae, M. gastri, M. triviale, M. haemophilum, M. africanum, M. thermoresistable* und *M*. *smegmatis.*

8. Verfahren nach einem der Ansprüche 1 bis 7, wobei die flüssige Probe wenigstens eine Probe ist, ausgewählt unter der Gruppe, bestehend aus Sputum, Spinalflüssigkeit, Faeces, Speichel, Blut, Geweben, Abstrichen, Flüssigkeit aus Magenspülung, Urin, Proben erhältlich aus Vorbehandlung dieser biologischen Proben, Wasser aus Bädern, Wasser aus Swimming Pools, Wasser aus Fischzuchten, Flusswasser, Seewasser und Meerwasser.

9. Verfahren nach einem der Ansprüche 1 bis 8, wobei die Menge der flüssigen Probe im Bereich von 50 µl bis 500 µl liegt.

10. Verfahren nach einem der Ansprüche 1 bis 8, wobei die Menge der flüssigen Probe im Bereich von 50 ml bis 200 ml liegt.

11. Arbeitsmittel zum Sammeln eines Mikroorganismus oder einer Zelle zur Verwendung in einem Verfahren nach Anspruch 1, umfassend ein Zentrifugationsrohr, wobei das Zentrifugationsrohr umfasst:
einen Filter, der den Innenraum des Zentrifugationsrohrs in eine obere Hälfte und eine untere Hälfte teilt; und
wasserabsorbierenden Harzpartikel, welche auf dem Filter angebracht sind.

12. Verfahren zur Amplifizierung oder spezifischen Detektion eines Gens eines Mikroorganismus oder einer Zelle, wobei man:
einen Mikroorganismus oder eine Zelle mit Hilfe eines Verfahrens nach einem der Ansprüche 1 bis 10 sammelt;
ein Gen aus dem Mikroorganismus oder der Zelle extrahiert, indem man eine Extraktionsreagenzlösung, enthaltend ein nichtionisches Detergens zu dem Mikroorganismus oder der Zelle gibt und das resultierende Gemisch erhitzt; und das extrahierte Gen amplifiziert oder spezifisch detektiert.

13. Verfahren nach Anspruch 12, wobei die Extraktionsreagenzlösung außerdem als Sammellösung dient.

14. Verfahren nach einem der Ansprüche 12 oder 13, wobei die Erhitzungstemperatur nicht niedriger als 70 °C und niedriger als 100°C ist.

15. Verfahren nach einem der Ansprüche 12 bis 14, wobei die Erhitzung 1 bis 30 Minuten durchgeführt wird.

16. Verfahren nach einem der Ansprüche 12 oder 13, wobei die Erhitzung 10 Minuten bei 96°C durchgeführt wird.

17. Verfahren nach einem der Ansprüche 12 bis 16, wobei der pH-Wert der Extraktionsreagenzlösung im Bereich von 7,0 bis 12,0 liegt.

18. Verfahren nach einem der Ansprüche 12 bis 17, wobei die Konzentration des nichtionischen Detergens in der Extraktionsreagenzlösung im Bereich von 0,01 bis 10 Gew.-% liegt.

19. Verfahren nach einem der Ansprüche 12 bis 18, wobei das nichtionische Detergens wenigstens eine Verbindung ist, die ausgewählt ist unter der Gruppe, bestehend aus D-Sorbitolfettsäureestern, Polyoxyethylenglykolsorbitanalkylestern und Polyoxyethylenglykol p-t-octylphenylethern.

20. Verfahren nach einem der Ansprüche 12 bis 19, wobei die Extraktionsreagenzlösung außerdem ein Metallchelat-bildendes Agens enthält.

21. Verfahren nach Anspruch 20, wobei die Konzentration des Metallchelatbildenden Agens in der Extraktionsreagenzlösung im Bereich von 0,1 bis 100 mM liegt.

22. Verfahren nach einem der Ansprüche 20 oder 21, wobei das Metallchelatbildende Agens wenigstens eine Verbindung ist, die ausgewählt ist unter der Gruppe, bestehend aus Ethylendiamintetraessigsäure (EDTA), Ethylenglykol bis(β-aminoethylether)-N,N,N',N'-tetraessigsäure (EGDTA), Diaminocyclohexantetraessigsäure, o-Phenanthrolin und Salicylsäure.

23. Verfahren nach einem der Ansprüche 12 bis 22, wobei das Gen mit Hilfe einer Polymerasekettenreaktion (PCR)-Methode amplifiziert oder spezifisch detektiert wird.

## Revendications

1. Procédé de prélèvement d'un micro-organisme ou d'une cellule d'un échantillon de liquide, comprenant :
la fourniture d'un tube de centrifugation comprenant un filtre sur lequel des particules de résine absorbant l'eau sont disposées, ledit filtre divisant un espace intérieur du tube en une partie supérieure et une partie inférieure, dans lequel l'échantillon liquide est versé dans le tube de centrifugation pour mettre l'échantillon liquide en contact avec la résine absorbant l'eau, de sorte qu'une partie de phase liquide de l'échantillon liquide est absorbée par la résine absorbant l'eau, et une solution de prélèvement est ensuite versée dans le tube de centrifugation pour mettre la solution de prélèvement en contact avec la résine absorbant l'eau, afin de prélever le micro-organisme ou la cellule piégés sur la surface de la résine absorbant l'eau dans la solution de prélèvement, et le tube de centrifugation est centrifugé, de sorte que la solution de prélèvement passe à travers le filtre pour se déplacer vers un fond du tube de centrifugation.

2. Procédé selon la revendication 1, dans lequel la centrifugation est réalisée entre 500 et 13000 g pendant 3 secondes à 60 minutes.

3. Procédé selon l'une quelconque des revendications 1 à 2, dans lequel une quantité de l'échantillon liquide ajouté n'est pas supérieure à une capacité d'absorption d'eau de la résine absorbant l'eau.

4. Procédé selon l'une quelconque des revendications 1 à 3, dans lequel la quantité de solution de prélèvement ajoutée est supérieure à une capacité d'absorption d'eau de la résine absorbant l'eau qui a absorbé la partie de phase liquide de l'échantillon liquide.

5. Procédé selon l'une quelconque des revendications 1 à 4, dans lequel la résine absorbant l'eau est un polymère réticulé hydrophile ayant un groupe fonctionnel hydrophile.

6. Procédé selon l'une quelconque des revendications 1 à 5, dans lequel le micro-organisme à collecter est au moins un élément choisi dans le groupe constitué de bactéries acido-résistants, de mycobactéries atypiques, de gonocoques, de bactéries de légionelle, de mycoplasmes, de spirochètes, de spirochètes de la syphilis, de chlamydiae, de rickettsie, de Mycobacterium leprae, de Spirillum minus, de staphylocoques, de streptocoques, d'Escherichia coli, de Pseudomonas aeruginosa, de Pasteurella pestis, de virus, de virus de l'encéphalite japonaise, de virus de l'hépatite B, de virus de l'hépatite C, d'ATLV, HIV et virus Ebola.

7. Procédé selon la revendication 6, dans lequel la bactérie acido-résistant est au moins une bactérie choisie dans le groupe constitué de M. avium, M. intracellularae, M. gordonae, M. tuberculosis, M. kansasii, M. fortuitum, M. chelonae, M. bovis, M. scrofulaceum, M. paratuberculosis, M. phlei, M. marinum, M. simiae, M. scrofulaceum, M. szulgai, M. leprae, M. xenopi, M. ulcerans, M. lepraemurium, M. flavescens, M. terrae, M. nonchromogenicum, M. malmoense, M. asiaticum, M. vaccae, M. gastri, M. triviale, M. haemophilum, M. africanum, M. thermoresistable, et M. smegmatis.

8. Procédé selon l'une quelconque des revendications 1 à 7, dans lequel l'échantillon liquide est au moins un échantillon choisi dans le groupe constitué de crachats, de fluide spinal, de fèces, de salive, de sang, de tissus, de tampons, de liquide obtenu par lavement, d'urine, d'échantillons obtenus par prétraitement de ces échantillons biologiques, d'eau dans les bains, d'eau dans les piscines, d'eau dans les aquariums, d'eau dans les rivières, d'eau de lac, et d'eau de mer.

9. Procédé selon l'une quelconque des revendications 1 à 8, dans lequel la quantité d'échantillon liquide est comprise entre 50 µl et 500 µl.

10. Procédé selon l'une quelconque des revendications 1 à 8, dans lequel la quantité d'échantillon liquide est comprise entre 50 ml et 200 ml.

11. Dispositif de prélèvement d'un micro-organisme ou d'une cellule utilisée pour le procédé selon la revendication 1, comprenant un tube de centrifugation,
le tube de centrifugation comprenant :
un filtre qui divise un espace intérieur du tube de centrifugation en une partie supérieure et une partie inférieure ; et
des particules de résine absorbant l'eau disposées sur le filtre.

12. Procédé d'amplification ou de détection spécifique d'un gène d'un micro-organisme ou d'une cellule, comprenant :
le prélèvement d'un micro-organisme ou d'une cellule par le procédé selon l'une quelconque des revendications 1 à 10 ;
l'extraction d'un gène du micro-organisme ou de la cellule en ajoutant une solution de réactif d'extraction contenant un détergent non-ionique au micro-organisme ou à la cellule et en chauffant le mélange obtenu ; et
l'amplification ou la détection spécifique du gène extrait.

13. Procédé selon la revendication 12, dans lequel la solution de réactif d'extraction sert également de solution de prélèvement.

14. Procédé selon les revendications 12 ou 13, dans lequel la température de chauffage n'est pas inférieure à 70°C et inférieure à 100°C.

15. Procédé selon l'une quelconque des revendications 12 à 14, dans lequel le chauffage est effectué pendant 1 à 30 minutes.

16. Procédé selon les revendications 12 ou 13, dans lequel le chauffage est effectué à 96°C pendant 10 minutes.

17. Procédé selon l'une quelconque des revendications 12 à 16, dans lequel un pH de la solution de réactif d'extraction est compris dans une gamme de 7,0 à 12,0.

18. Procédé selon l'une quelconque des revendications 12 à 17, dans lequel une concentration du détergent non-ionique dans la solution de réactif d'extraction est comprise entre 0,01 à 10% en poids.

19. Procédé selon l'une quelconque des revendications 12 à 18, dans lequel le détergent non-ionique est au moins un choisi dans le groupe constitué d'esters d'acide gras de D-sorbitol, d'alkyl-esters de sorbitanne de polyoxyéthylène-glycol, et de polyoxyéthylène-glycol p-t-octylphényl-éthers.

20. Procédé selon l'une quelconque des revendications 12 à 19, dans lequel la solution de réactif d'extraction contient en outre un agent chélatant métallique.

21. Procédé selon la revendication 20, dans lequel une concentration de l'agent chélatant métallique dans la solution de réactif d'extraction est de 0,1 à 100 mM.

22. Procédé selon la revendication 20 ou 21, dans lequel l'agent chélatant métallique est au moins un élément choisi dans le groupe constitué d'acide éthylènediaminetétraacétique (EDTA), d'acide éthylène glycol bis(β-aminoéthyl-éther)-N,N,N',N'-tétraacétique (EGTA), d'acide diaminocyclohexane tétraacétique, d'o-phénanthroline, et d'acide salicylique.

23. Procédé selon l'une quelconque des revendications 12 à 22, dans lequel le gène est amplifié ou détecté spécifiquement par un procédé de réaction en chaîne de polymérase (PCR).
